Europäisches Patentamt

European Patent Office

Office européen des brevets

(11) Veröffentlichungsnummer: **0 286 731**
**A1**

# EUROPÄISCHE PATENTANMELDUNG

(21) Anmeldenummer: **87118563.3**

(22) Anmeldetag: **15.12.87**

(51) Int. Cl.⁴: **A61B 5/03**

(30) Priorität: **03.01.87 HU 1287**

(43) Veröffentlichungstag der Anmeldung:
**19.10.88 Patentblatt 88/42**

(84) Benannte Vertragsstaaten:
**AT BE CH DE ES FR GB GR IT LI LU NL SE**

(71) Anmelder: **Mikroker, Szervezö Közvetitö
és Ipari Kisszövetkezet 27, Szondy utca
H-1204 Budapest(HU)**

(72) Erfinder: **Török, Miklos, Dr.
Komocsi u. 21
HU-1145 Budapest(HU)**
Erfinder: **Boda, Janos
Kruper u. 4
HU-1111 Budapest(HU)**

(74) Vertreter: **Patentanwälte Viering & Jentschura
Steinsdorfstrasse 6
D-8000 München 22(DE)**

(54) **Verfahren und Gerät zum externen Messen von Gebärmutterkontraktionen.**

(57) Verfahren und Gerät zum externen Messen von Gebärmutterkontraktionen, insbesondere zum Anzeigen einer drohenden Frühgeburt. Bei dem Verfahren werden die Anzahl und Intensität der Gebärmutterkontraktionen zweckmäßig periodisch abgetastet. Die abgetasteten analogen Signale werden gegebenenfalls nach Verstärkung in digitale Signale umgewandelt, wonach die digitalen Signale gespeichert und verarbeitet, zuletzt angezeigt und/oder an einen Mikroprozessor weitergeleitet werden. Während der Dauer der Messung werden der Größe und Dauer der abgetasteten Gebärmutterkontraktion entsprechende Werte mit einer im voraus bestimmten Schwellengröße und ein vorbestimmtes Zeitintervall charakterisierenden Werten verglichen, und die über der Schwellengröße liegenden und gleichzeitig in das Intervall entfallenden Meßwerte mit den zugehörigen Zeitpunkten gespeichert, wonach die Messung wiederholt wird und die Anzahl der während einer gegebenen Zeitspanne gespeicherten Meßwerte bestimmt wird und, falls die Anzahl der gespeicherten Meßwerte einen im voraus eingestellten Wert überschreitet, ein Alarmsignal erzeugt wird. Das Gerät enthält eine Zentraleinheit (1), einen an die Zentraleinheit über einem inneren Systembus (23) angeschlossenen Arbeitsspeicher (3), einen Datenspeicher (2), eine oder mehrere Anpaßeinheiten sowie eine analoge Meßeinheit (24). Die analoge Meßeinheit (24) enthält einen Meßumformer (10), einen an den Ausgang des Meßumwandlers über einen Tiefpaßfilter (9) angeschlossenen Verstärker (8) und gegebenenfalls einen mit dessen Ausgang verbundenen Komparator (6). Der Ausgang des Verstärkers (8) ist über einen A/D-Umwandler (5) mit dem inneren Systembus (25) verbunden. Desweiteren ist als Anpaßenheit wenigstens eine Zweiwege-Kopplungseinheit (4) vorgesehen, die über einen Tonfrequenzstromkreis (13) mit einer Anzeige (22) verbunden ist.

Fig.1

## Verfahren und Gerät zum externen Messen von Gebärmutterkontraktionen

Die Erfindung betrifft ein Verfahren zum externen Messen von Gebärmutterkontraktionen, insbesondere zum frühzeitigen Anzeigen einer drohenden Frühgeburt, in dessen Verlauf die Anzahl und die Intensität der Gebärmutterkontraktionen zweckmäßig periodisch abgestastet werden, wonach die abgetasteten analogen Signale gegebenenfalls nach Verstärkung in digitale Signale umgewandelt werden; die digitalen Signale werden gespeichert, verarbeitet und zuletzt angezeigt und/oder an einen Rechner weitergeleitet. Die Erfindung betrifft auch ein zur Durchführung des erfindungsgemäßen Verfahrens dienendes Gerät.

Das Geburtsproblem unserer Zeiten besteht in der Herabsetzung der Sterblichkeitsrate in Neugsborenenalter. Aufgrund der in mehreren Richtungen vorgenommenen Analyse der Frage ist est schon heute eindeutig, daß die Sterblichkeit im Neugeborenenalter mit der Häufigkeit der Frühgeburten zusammenhängt.

Im Interesse der präventiven Verhütung der Frühgeburten war man durch die Analyse von Sammelstatistiken und Fragebögen bestrebt, die erhöht frühgeburtgefährdeten Personen unter den - schwangeren Frauen herauszufinden und die Frühgeburt durch Versetzen in den Krankenstand, durch medikamentöse Behandlung und durch eventuelle Aufnahme in das Krankenhaus zu verhindern.

Der Nachteil der Methode besteht darin, daß ein hoher Aufwand an Personen und finanziellen Mitteln erforderlich ist; gleichzeitig ist "die Treffsicherheit" keinesfalls befriedigend; viele der später frühgebärenden schwangeren Frau erhalten die erhöhte Fürsorge nicht und für eine große Anzahl von Frauen ist die erhöhte Fürsorge überflüssig.

Ein grundsätzliches Problem besteht darin, daß bisher keine solche Methode zur Verfügung steht, durch die die drohende Frühgeburt vorzeitig erkannt werden könnte und in dieser Weise diejeinigen Schwangeren gefunden werden könnten, bei denen eine Frühgeburt zu erwarten ist.

Man hat schon früh beobachtet, daß die Gebärmutterkontraktionen während der ganzen Dauer der Schwangerschaft kontinuierlich abgetastet werden können. Wie M. Katz, P.B. Gill und R.B. Newman in der Ausgabe 154/1986 der Zeitschrift "American Journal of Obstretics and Gynaecology" ausführlich beschrieben, können zwischen den Gruppen der Frühgebärenden und der Normalgebärenden beträchtlicht Unterschiede in der Intensität und Häufigkeit der Gebärmutterkontraktionen während der Schwangerschaft festgestellt werden. Während der ganzen Dauer der in einer Frühgeburt endenden Schwangerschaften sind Häufigkeit und Intensität der Gebärmutterkontraktionen bedeutend größer als bei denjenigen, bei denen die Schwangerschaft nicht in einer Frühgeburt endet. Gleicherweise kann bewiesen werden, daß die Aktivität der Gebärmutter in den Tagen vor der Geburt verstärkt wird, was darauf hinweist, daß die Geburt voraussichtlich einsetzt.

Die gemeinsame Charakteristik der bekannten zur Messung der Gebärmutteraktivität dienenden Methoden besteht darin, daß auf die Bauchwand der Mutter von außen ein Druckumformer (ein sogenannter Dehnungsmeßstreifen) gelegt wird. Das analoge Signal des Umformers wird verstärkt und elekontrisch verarbeitet und z. B. in Form eines Diagramms für den Arzt angezeigt.

Obwohol das Resultat sofort ausgewertet werden kann - aber nur für den Fachmann - ermöglicht die Untersuchungsmethode die Massenauswahl der schwangeren Mütter überhaupt nicht. Ein weiter Nachteil besteht darin, daß eine großdimensionierte Vorrichtung beansprucht wird, die nur zur Anwendung in der Klinik und ärtzlich überwacht geeignet ist; gleichzeitig ist die Meßdauer ziemlich kurz.

Eine weiterentwickelte Lösung stellt das wehenregistrierende und analysierende Gerät dar, das von M. Katz und P.J. Gill in Nummer 66/1983 der Zeitschrift "American Journal of Obstetrics and Gynaecology" beschrieben wird. Es handelt sich um ein tragbares Gerät, das im Heim der Kranken eingesetzt werden kann. Das Gerät besteht aus zwei verschiedenen Grundeinheiten, von denen die eine Einheit die Wehentätigkeit der Gebärmutter in traditioneller Weise 200 Minuten lang registriert; die Daten aus den 200 Minuten werden gespeichert und in 6 - 8 Minuten über eine Fernsprechlinie in den auf dem Telefonapparat des Arztes montierten Empfängerapparat weitergeleitet, wo die Daten in Form einer Wehenkurve erscheinen. Der Arzt analysiert die Daten kontinuierlich, im Bedarfsfall wird die Patientin telefonisch ins Spital beordert.

Der Nachteil der Lösung besteht darin, daß sie eine gut entwickelte Infrastruktur, ein ausgebautes gut funktionierendes Telefonnetz und eine 24stündige ständige ärtzliche Inspektion beansprucht. Trotz der tragbaren Ausführungsform ist das Gerät großdimensioniert; desweiteren sind ein an das Telefon angepaßter Sender und eine auswertende Empfängereinheit erforderlich, die das System komplizieren und die Kosten erhöhen.

Der Erfindung wurde das Ziel gesetzt, ein Verfahren und ein Gerät zu entwickeln, die von der - schwangeren Frau selbst zur Messung der Wehentätigkeit der Gebärmutter geeignet sind, wobei die Meßergebnisse gespeichert und nach Bedarf angezeigt werden können und bei einer auf zu erwartende Frühgeburt hinweisenden Gebärmuttertätigkeit ein Warnsignal abgegeben wird. Zu der Zielsetzung gehört noch die Forderung, daß das Gerät kleindimensioniert, einfach aufgebaut und wirtschaftlich erzeugbar ist.

Der Erfindungsgedanke beruht auf der Erkenntnis, daß die die Gebärmutteraktivität charakerisierende Wehenkurve in mathematischer Form abgefaßt werden kann; in dieser Wesie können die dem eingegebenen Programm entsprechend erscheinenden, auf eine drohende Frühgeburt hinweisenden, auf eine Zeiteinheit entfallenden Wehen gezählt werden und ein die schwangere Frau alarmierendes Signal erzeugt werden.

Zu der Erkenntnis gehört noch die Tatsache, daß das der Zielsetzung entsprechende Gerät mit einem von einem Mikroprozessor kontrollierten Meß-und Signalverarbeitungsstromkreis mit niedrigem Verbrauch ausgestaltet werden kann.

Dem gesetzten Ziel entsprechend dient das erfindungsgemäße Verfahren zur äußerlichen Messung der Gebärmutterkontraktionen, insbesondere zum frühzeitigen Anzeigen einer drohenden Frühgeburt, bei dem die Anzahl und Intensität der Gebärmutterkontraktionen zweckmäßig periodisch abgetastet werden, die abgetasteten analogen Signale gegebenenfalls nach Verstärkung in digitale Signale umgewandelt werden die digitalen Signale gespeichert werden und zuletzt angezeigt und/oder einem Mikroprozessor zugeleitet werden. Das erfindungsgemäße Verfahren beruht darauf, daß während der Dauer des Messung die der Größe und Zeitdauer der abgetasteten Gebärmutterkontraktionen entsprechenden Werte mit für eine vorbestimmte Schwellengröße und ein Zeitintervall charakterisierenden Werten verglichen werden un die über der Schwellengröße liegenden und gleichzeitig in das Zeitintervall fallenden Werte mit deren Zeitpunkt gespeichert werden, wonach die Messung wiederholt wird und dabei die Anzahl der während einer vorgegebenen Zeitspanne gespeicherten Daten bestimmt wird, und falls die Anzahl der Daten einen vorbestimmten Wert überschreitet, ein Warnsignal erzeugt wird.

Eine weitere Charakteristik der Erfindung kann darin bestehen, daß die Zeitdauer der Messungen zweckmäßig 600 s beträgt und im Verlauf der Verarbeitung diejenigen Gebärmutterkontraktionen in Betracht gezogen werden, die in den Bereich zwischen 30 und 300 s fallen, wobei das Alarmsignal dann erzeugt wird, wenn die gespeicherten Gebärmutterkontraktionen in ihrer Anzahl einen bestimmten Wert überschreiten.

Bei einer zweckmäßigen Verwircklichung des erfindungsgemäßen Verfahrens werden auch die Fläche unter der die Gebärmutterkontraktionen charakterisierenden Kurve, sowie von Fall zu Fall die Funktion der Amplitudenverteilung bestimmt.

Bei einer anderen Version des erfindungsgemäßen Verfahrens werden die Gebärmutterkontraktionen 24 Stunden lang kontinuierlich oder in dreistündigen Intervallen achtmal, z. B. über 8 Tage hin, oder in einstündigen Abständen 24mal, z. B. über 24 Tage hin gemessen.

Bei allen Versionen des Verfahrens werden das Anzeigen und/oder Weiterleiten der Daten am Ende der jeweiligen Messung auf die Durchführung der arithmetischen Befehle folgend durchgeführt.

Das erfindungsgemäße Gerät dient zur äußerlichen Messung der Gebärmutterkontraktionen, insbesondere zum frühzeitigen Anzeigen einer drohenden Frühgeburt, und enthält eine Zentraleinheit, einen operativen, mit der Zentraleinheit über einen inneren Systembus verbundenen Speicher, einen Datenspeicher und eine oder mehrere Anpaßeinheiten sowie eine analoge Meßeinheit, und ist so ausgestaltet, daß die analoge Meßeinheit einen Meßwandler, einen an den Ausgang des Meßwandlers über einen Tiefpaßfilter angeschlossenen Verstärker und gegebenenfalls einen an dessen Ausgang angeschlossenen Komparator enthält. Der Ausgang des Verstärkers ist über einen A/D-Umwandler mit dem inneren Sytembus verbunden; desweiteren ist je Anpaßeinheit wengistens eine Zweiwege-Kopplungseinheit, ein schallabgebendes Mittel, eine Tastatur und über den Anzeigesteuerstromkreis ein Anzeigegerät angeschlossen.

Das Gerät kann desweiteren auch dadurch gekennzeichnet werden, daß der Meßwandler ein piezoelektrischer Druckumformer ist, der Komparator mit einer den unteren und oberen Grenzwert anzeigenden einstellbaren Anzeigevorrichtung, zweckmäßig mit zwei LED-Anzeigevorrichtungen versehen ist.

Bei einer zweckmäßigen Ausführungsform sind die Zentraleinheit, die Zweiweg-Kopplungseinheit sowie der Taktsignalstromkreis in dem Mikroprozessor enthalten; zusätzlich zu der Zweiweg-Kopplungseinheit als Anpaßeinheit ist der Mikroprozessor mit einem Reihensignalein-und -ausgang, zweckmäßig mit einem RS-232 Koppler, und mit einem 8-Bit-Parallelsignalausgang versehen.

Bei einer anderen vorteilhaften Ausführungsform ist die Tastatur eine Folientastatur, als Anzeiger dient ein LCD-Display mit 7 x 7 Digit, während als tongebendes Mittel ein Miniaturlautsprecher oder ein keramischer Strahler dient.

Bei einer weiteren vorteilhaften Ausführungsform bildet eine Akkumlatorbatterie die Speisespannungsquelle des Geräts, die über eine stabilisierte Speiseeinheit und einen die Batterie kontrolierenden Stromkreis an die sonstigen Stromkreise angeschlossen ist, während zum Speisen der Anzeigeeinheit ein Spannungsunwandler vorgesehen ist.

Bei allen der erwähnten Ausführungsformen ist das Gerät in einem vorteilhaft aus Kunststoff gefertigten tragbaren Gehäuse angeordnet, an welchen ein Tragriemen befestigt ist, der zur Befestigung auf dem Körper der schwangeren Mutter und zur elektronischen Nullierung geeignet ist.

Das erfindungsgemäße Verfahren und das zur Durchführung des Verfahrens dienende Gerät weisen zahlreiche vorteilhafte Eigenschaften auf. Von denen ist die wesentlichste, daß die Möglichkeit einer Frühgeburt im Heim der schwangeren Frau ohne die Anwesenheit eines Fachmanns mit höherer Sicherheit angezeigt werden kann.

Ein weiterer Vorteil zeigt sich darin, daß die Dienstleistungen seitens des Facharztes frei gewählt werden können, das Meßergebnis kann unmittelbar von der eingebauten Anzeigevorrichtung abgelesen werden oder es kann über zweckdienlich gewählten Kopplungseinheiten zu ausgewählten externen Einrichtungen geleitet werden.

Es wird als vorteilhaft betrachtet, daß das Gerät unabhängig von der Infrastruktur der Umgebung, z. B. von der Versorgung mit Telefon, gebraucht werden kann. Das Gerät ist kleindimensioniert und leicht; so verursacht es keine Unbequemlichkeit für die schwangere Mutter. Die Bedienung ist einfach und infolge der angewendeten Stromkreislösungen kann das Gerät mit einer Batterie langfristig benutzt werden.

Die Erfindung wird anhand eines Ausführungsbeipiels mit Hilfe der Zeichnungen näher erläutert; in der Zeichnung zeigen:

Figur 1 das Blockschema des erfindungsgemäßen Gerätes,

Figur 2 die Draufsicht einer Ausführungsform des erfindungsgemäßen Gerätes und

Figur 3 die Seitenansicht des Gerätes nach Figur 2.

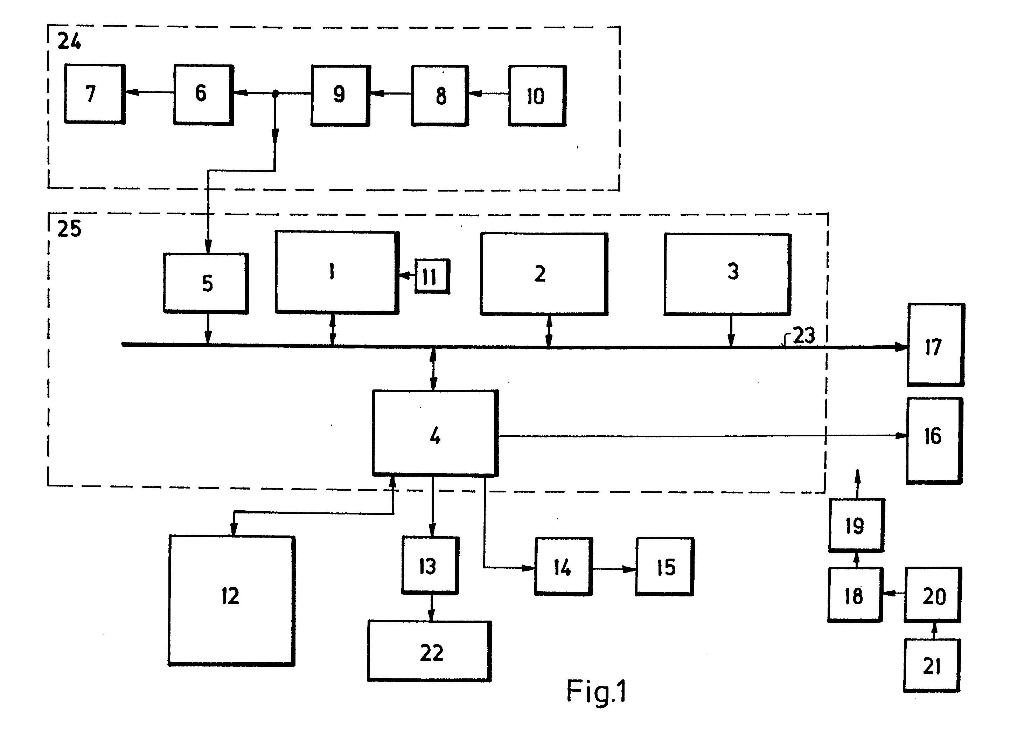

Figur 1 zeigt das Blockschema des erfindungsgemäßen Gerätes. Die beiden Hauptteile des Gerätes sind die analoge Meßeinheit 24 und die von der Zentraleinheit 1 gesteuerte digitale Signalempfängereinheit 25.

Der Meßumformer 10 bildet den Eingang der analogen Meßeinheit 24, der zweckmäßig ein hochempfindlicher temperaturkompensierter piezoelektrischer Druckumformer ist. Der Ausgang der Meßumformers 10 ist über den Tiefpaßfilter 8 an den Eingang des Verstärkers 9 angeschlossen. Der Ausgang des Verstärkers 9 ist teilsweise mit dem A/D Umwandler 5, teilweise mit dem Eingang des Komparators 6 verbunden.

Der Komparator 6 enthält einen Komparator für den unteren Grenzwert und einen Komparator für den oberen Grenzwert, sowie Anzeigevorrichtungen 7, zweckmäßig zwei LED-Anzeigevorrichtungen, die den unteren bzw. oberen Grenzwert anzeigen.

Damit die gewünschte Genauigkeit erreicht werden kann, ist der 8-Bit-Ausgang des A/D-Umwandlers 5 an den internen Bus 23 der digitalen signalverarbeitenden Einheit 25 angeschlossen.

An den Bus 23 sind die Elemente der digitalen signalverarbeitenden Einheit 25 angeschlossen, so die Zentraleinheit 1, der Datenspeicher 3, der Arbeitsspeicher 3 und die Zweiwege-Koppeleinheit 4 sowie der parallele Aus-/Eingang 17.

Bei dem hier geschilderten Ausführungsbeispiel ist die Zentraleinheit 1 ein 8-Bit-Mikroprozessor, der Datenspeicher ein 16-Kbyte-RAM, der Arbeitsspeicher 3 ein EPROM mit 16 Kbyte-Kapazität. Zwecks Sicherstellung eines geringen Verbrauchs weisen die Schaltungselemente einen CMOS-Aufbau auf, vorteilhaft können z. b. integrierte Stromkreise des Typs NSC 800, NSC 810, NSC 830 verwendet werden, die die Zentraleinheit 1, die Zweiwege-Kopplungseinheit 4 und den Taktsignalstromkreis 22 enthalten.

Die Zweiwege-Kopplungseinheit 4 ist mit den zur Kommunikation mit dem Gerät geeigneten Stromkreisen verbunden, so mit dem Reihen-Aus-/Eingang 10, der Tastatur 12 sowie dem Steuerstromkreis 13 für die Anzeigevorrichtung. Der Reihen-Aus-/Eingang 16 wird zweckmäßig von einer standarisierten RS-232 Kopplung gebildet, während die Tastatur 12 vorteilhaft eine Folientastatur ist. An dem Ausgang des Treiberstromkreises 12 der Anzeigevorrichtung ist die Anzeigevorrichtung 22 angschlossen, die in unserem Beispiel ein LCD-Display mit 7 x 7 Digit ist.

An den weiteren Ausgang der Zweiwege-Kopplungseinheit 4 ist der Tonfrequenzstromkreis 14 angeschlosen, der mit dem tongebenden Mittel 15, z. B. einem Miniaturlautsprecher oder einem keramischen Strahler verbunden ist.

Zur Speisespannung des Gerätes dient die aufladbare Akkumulatorbatterie, die über die stabilisierte Speiseeinheit 20 und den die Batterie kontrollierenden Stromkreis 19 mit den sonstigen Stromkreisen verbunden ist. Der Speiseeinheit 20 ist ein Spannungsumwandler 18 zugeordnet, dessen Ausgang mit dem Steuerstromkreis 13 für die Anzeigevorrichtung verbunden ist.

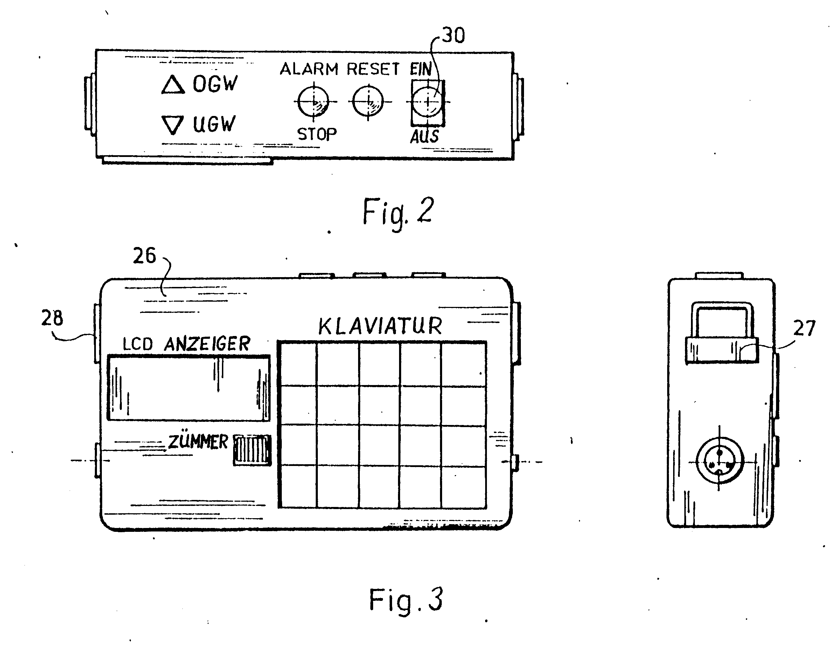

Figur 2 und 3 veranschaulichen den mechanischen Aufbau des Gerätes. Vorteilhaft weist des Gerät ein Gerätgehäuse 26 aus Kunststoff auf. An der Stirnplatte des Gerätegehäuse 26 sind das Anzeigefeld 22, die Tastatur 12 und die Bedientasten des tongebenden Mittels 15 zu finden. Auf der unteren, dem Mutterkörper zugewandten Platte des Gerätegehäuses 26 ist der Meßumformer 10 montiert. Die beiden LED-Anzeigen 7 sind für die schwangere Mutter gut sichtbar angeordnet. An der Seitenwand des Gerätegehäuses 16 sind der Ein- und Ausschalter 30 sowie der Startknopf 31 angeordnet.

An dem Gerätegehäuse 26 ist mit den Stellelementen 28 ein Tragriemen 27 befestigt, der zur Befestigung an dem Mutterkörper und zur elektronischen Nullierung geeignet ist.

Die Wirkungsweise des obenbeschriebenen Gerätes zur Realisierung des erfindungsgemäßen Verfahrens ist wie folgt:

Die schwangere Mutter legt das das Gerät enthaltende Gerätegehäuse 26 auf ihren Bauch; nach erfolgter Einschaltung wird der Tragriemen 27 solange mit Hilfe der Stellelemente 28 angezogen oder gelockert, bis die optischen Signale der beiden LED-Anzeigen 7 erlöschen.

Durch Drücken des Startknopfs 31 wird das Meßverfahren gestartet, welches gemäß dem in dem Arbeitsspeicher 3 gespeicherten Programm abläuft. Das die Wehenaktivität der Gerbärmutter charakterisierende kontinuierliche, quasi periodische Gleichspannungssignal, das sich zeitlich sehr langsam ändert, wird von dem Meßumformer 10 geliefert. Der Tiefpaßfilter 9 filtert die Störsignale aus, während der Versträker 8 die Signale mit dem niedrigen Pegel auf den zur Betätigung des A/D-Umwandlers 5 erforderlichen Pegel verstärkt.

Der A/D-Umwandler 5 wandelt das verstärkte analoge Signal des Meßumformers 10 durch Sampling in eine binäre Information um, die in den Systembus 23 weitergeleitet wird.

Die binäre Information wird mit den zu den aktuellen Messungen gehörenden Zeitpunkten zusammen in dem Datenspeicher 2 gespeichert. Im Laufe der Signalverarbeitung werden diese Daten mit den gesetzten Daten in dem Arbeitsspeicher 3 verglichen; simultan werden auch das digitale Filtrieren und Prüfen der Glaubbarkeit durchgeführt.

Das digitale Filtrieren erlaubt, daß das Gerät ausschließlich die Signale, die im Bereich zwischen den angegebenen Grenzenwerten liegen, und die dazugehörenden Zeitpunkte speichert, wobei die Daten bis zum Abfragebefehl oder bis zum Vorhandensein der Speisespannung gespeichert werden.

Die digitale Signalverarbeitung wird anhand eines kontreten Meßprozesses näher erläutert. Nachdem das Meßprogramm gestartet worden ist, werden die Messungen 600 s lang fortgesetzt, wonach die während dieser Zeitspanne gewonnen Meßergebnisse verarbeitet werden. Diejenigen Daten werden ausgewählt, bei denen die Zeitdauer der Gebärmutterkontraktionen in den Bereich zwischen 30 und 300 s fällt und die Größe den Grenzwert der Schwellenspannung überschreitet. In dem nächsten Arbeitsschritt werden die die Größe und Zeitdauer der Gebärmutterkontraktionen betreffenden Daten gespeichert, die eventuellen peripherialen Arbeitsgänge werden durchgeführt. Nun besteht die Möglichkeit zur Anzeige der gespeicherten Daten auf der Anzeige 22 oder zur Weiterleitung über eine der Kopplungen.

Nach Beendigung der peripherialen Arbeitsgänge werden die zwischen den einzelnen gespeicherten, die Gebärmutterkontraktionen charakterisierenden Daten abgelaufenen Zeitspannen überprüft und wenn die Anzahl der Gebärmutterkontraktionen je Stunde mehr als vier ist, wird ein akustisches Warnsignal erzeugt. Beim eventuellen Schadhaftwerden des Gerätes wird gleicherweise ein akustisches Signal abgegeben. Bei einem Alarmsignal muß die schwangere Mutter sofort ihren behandelnden Arzt alarmieren.

Die Messung der Gebärmutterkontraktionen wird aufgrund mehrerer Programme in einer dem Zustand der schwangeren Mutter am besten entsprechende Weise vorgenommen.

So besteht die Möglichkeit 24 Stunden lang kontinuierlich zu messen, oder 8mal in dreistündigen Etappen, z.B. 8 Tage lang, oder in einstündigen Etappen, z.B. 24 Tage lang.

Das Gerät bietet die Möglichkeit, die Fläche unter der die einzelnen Gebärmutterkontraktionen kennzeichnenden Kurve oder die Funktion der Amplitundenverteilung zu statistichen Zwecken zu bestimmen.

Das erfindungsgemäßen Gerät kann vorteilhaft während der Schwangerenversorgung zur Voranzeige einer eventuellen Frühgeburt mit voller Sicherheit verwendet werden, wodurch die schädlichen Auswirkungen der Frühgeburt eliminiert werden.

## Ansprüche

1. Verfahren zum externen Messen von Gebärmutterkontraktionen, insbesondere zum Anzeigen einer drohenden Frühgeburt, bei dem die Anzahl und die Intensität der Gebärmutterkontraktionen zweckmäßig periodisch abgetastet werden, die agbetasteten analogen Signale gegebenenfalls nach Verstärkung in digitale Signale umgewandelt werden, und die digitalen Signale gespeichert und verarbeitet, zuletzt angezeigt und/oder an einen Mikroprozessor weitergeleitet werden, dadurch gekennzeichnet, daß während der Meßdauer der Größe und Dauer der abgestasten Gebärmutterkontraktionen entsprechenden Werte mit einer im voraus bestimmten Schwellengröße und einem vorbestimmten Zeitintervall verglichen werden und die über der Schwellengröße liegenden und gleichzeitig in das Intervall fallenden Werte mit den zugehörigen Zeitpunkten gespeichert werden, wonach die Messung wiederholt wird und die Anzahl der während einer gegebenen Zeitspanne gespeicherten Daten bestimmt wird, und falls die Anzahl der gespeicherten Daten einen im voraus eingestellten Wert überschreitet, ein Alarmsignal erzeugt wird.

2. Verfahren nach Anspruch 1, dadurch gekennzeichnet, daß die Dauer der Messungen zweckmäßig 600 s beträgt, im Verlauf der Verarbeitung die in den Bereich zwischen 30 und 300 s entfallenden Gebärmutterkontraktionen berücksichtigt werden und das Alarmsignal erst dann erzeugt wird, wenn die Anzahl der Gebärmutterkontraktionen einen bestimmten Wert übertrifft.

3. Verfahren nach Anspruch 2 oder 2, dadurch gekennzeichnet, daß die Fläche unter der die einzelnen Gebärmutterkontraktionen charakterisierenden Kurve sowie von Fall zu Fall auch die Funktion der Amplitudenverteilung bestimmt werden.

4. Verfahren nach einem der Ansprüche 1 bis 3, dadurch gekennzeichnet, daß die Gebärmutterkontraktionen 24 Stunden lang kontinuierlich oder in dreistündigen Etappen achtmal z. B. 24 Tage lang gemessen werden.

5. Verfahren nach einem der Ansprüche 1 bis 4, dadurch gekennzeichnet, daß die Daten nach Beendigung der Messung und Ausführung der arithmetischen Befehle angezeigt und/oder weitergeleitet werden.

6. Gerät zur externen Messung von Gebärmutterkontraktionen, insbesondere zum frühzeitigen Anzeigen einer drohenden Frühgeburt, mit einer Zentraleinheit (1), einem an die Zentraleinheit über einen inneren Systembus (23) angeschlossenen Arbeitsspeicher (3), einem Datenspeicher (2), einer oder mehreren Anpaßeinheiten sowie einer analogen Meßeinheit (24), dadurch gekennzeichnet, daß die analoge Meßeinheit (24) einem Meßumformer (10), einen an den Ausgang des Meßumformers (10) über einen Tiefpaßfilter (9) angeschlossenen Verstärker (8) und gegebenenfalls einen mit dessen Ausgang verbundenen Komparator (6) enthält, der Ausgang des Verstärkers (8) über einen A/D-Umwandler (5) mit dem inneren Systembus (23) verbunden ist und es mit wenigstens einer Zweiwege-Kopplungseinheit (4) als Anpaßeinheit versehen ist und die Zweiwege-Kopplungseinheit (4) über einen Tonfrequenzstromkreis (14) mit einem tongebenden Mittel (15) und einer Tastatur (12) und über einen Anzeigesteuerstromkreis (13) mit einer Anzeige (22) verbunden ist.

7. Gerät nach Anspruch 6, dadurch gekennzeichnet, daß ein piezoelektrischer Druckumformer den Meßumformer (10) bildet und der Komparator (6) mit einer einstellbaren, den oberen und den unteren Grenzwert anzeigenden Anzeige (7) ergänzt ist, die zweckdienlich aus zwei LED-Anzeigen gebildet ist.

8. Gerät nach Anspruch 6 oder 7, dadurch gekennzeichnet, daß die Zentraleinheit (1), die Zweiwege-Kopplungseinheit (4) sowie der Taktsignalstromkreis (11) in einem Mikroprozessor (25) enthalten sind.

9. Gerät nach einem der Ansprüche 6 bis 8, dadurch gekennzeichnet, daß es neben der Zweiwege-Kopplungseinheit (4) als Anpaßeinheit mit einen Reihen--Signalaus- -eingang (16), zweckmäßig mit einer RS-232 Kopplung und einem 8-Bit-Paralellausgang (7) versehen ist.

10. Gerät nach einem der Ansprüche 6 bis 9, dadurch gekennzeichnet, daß die Tastatur (12) vorteilhaft eine Folientastatur ist, als Anzeiger (22) ein LCD-Display mit 7 x 7 Digit dient und als tongebendes Mittel ein Miniaturlautsprecher oder ein keramischer Strahler eingesetzt wird.

11. Gerät nach einem der Ansprüche 6 bis 10, dadurch gekennzeichnet, daß die Speisespannungsquelle eine aufladbare Akkumulatorbatterie (21) ist, die über eine stabilisierte Speiseeinheit (20) und einen die Batterie kontrollierenden Stromkreis (19) an die sonstigen Stromkreise angeschlossen ist, während zur Speiseversorgung der Anzeige (22) ein Spannungsumwandler (18) vorhanden ist.

12. Gerät nach einem der Ansprüche 6 bis 11, dadurch gekennzeichnet, daß das Gerät in einem tragbaren, vorteilhaft aus Kunststoff gefertigten Gerätegehäuse (26) angeordnet ist, an dem ein Tragriemen (27) angeschlossen ist, der über Stellelemente (23) zur Befestigung auf dem Mutterkörper und zur elektronischen Nullierung geeignet ist.

Fig.1

0 286 731

△ OGW

▽ UGW

ALARM RESET EIN
30

STOP

AUS

## Fig. 2

26

28

LCD ANZEIGER

KLAVIATUR

ZÜMMER

27

## Fig. 3

0 286 731

Europäisches Patentamt

**EUROPÄISCHER RECHERCHENBERICHT**

Nummer der Anmeldung

EP 87 11 8563

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 060 891 (KOIE ELECTRONICS IND. CO.) <br> * Zusammenfassung; Seite 1, Zeilen 70-108 * <br> --- | 1,5 | A 61 B 5/0 |
| X | FR-A-2 403 058 (DITTMAR) <br> * Seite 3, Zeile 39 - Seite 4, Zeile 19; Seite 7, Zeile 24 - Seite 8, Zeile 19; Figuren 1,2 * <br> --- | 6-12 | |
| A | US-A-3 989 034 (HOJAIBAN) <br> * Zusammenfassung * <br> --- | 1,6 | |
| A | WO-A-8 603 115 (CLARE) <br> * Zusammenfassung; Figur 8 * <br> --- | 1 | |
| A | BIOMEDICAL ENGINEERING, Band 11, Nr. 11, November 1976, Seiten 378-379, United Trade Press Ltd, London, GB; C.C. BASHFORD et al.: "A method for monitoring uterine activity in induced labour" <br> * Figur 1 * <br> --- | 3 | RECHERCHIERTE SACHGEBIETE (Int. Cl.4) <br><br> A 61 B |
| D,A | AMERICAN JOURNAL OF OBSTRETICS AND GYNAECOLOGY, Band 154, Juni 1986, Seiten 1253-1256; M. KATZ et al.: "Detection of preterm labor by ambulatory monitoring of uterine activity for the management of oral tocolysis" <br> * Zusammenfassung * <br> ----- | 1,2,4 | |

Der vorliegende Recherchenbericht wurde für alle Patentansprüche erstellt

| Recherchenort | Abschlußdatum der Recherche | Prüfer |
|---|---|---|
| DEN HAAG | 28-03-1988 | CHOWDHURY |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..............................................................................
& : Mitglied der gleichen Patentfamilie, übereinstimmendes Dokument

EPO FORM 1503 03.82 (P0403)